(19) Europäisches Patentamt
European Patent Office
Office européen des brevets

(11) Veröffentlichungsnummer: **0 003 766**
**A1**

(12) # EUROPÄISCHE PATENTANMELDUNG

(21) Anmeldenummer: 79100340.3

(22) Anmeldetag: 06.02.79

(51) Int. Cl.$^2$: C 07 C 127/22
A 01 N 9/20

(30) Priorität: 14.02.78 DE 2806213

(43) Veröffentlichungstag der Anmeldung: 05.09.79 Patentblatt 79/18

(84) Benannte Vertragsstaaten: BE CH DE FR GB IT NL SE

(71) Anmelder: **Bayer Aktiengesellschaft**
**Zentralbereich Patente,Marken und Lizenzen Bayerwerk**
**D-5090 Leverkusen 1(DE)**

(72) Erfinder: **Sirrenberg, Wilhelm, Dr.**
**Wuppertaler Strasse 21**
**D-4322 Sprockhövel(DE)**

(72) Erfinder: **Klauke, Erich, Dr.**
**Eichendorffweg 8**
**D-5063 Odenthal - Hahnenberg(DE)**

(72) Erfinder: **Brandes, Wilhelm, Dr.**
**Eichendorffstrasse 3**
**D-5653 Leichlingen(DE)**

(72) Erfinder: **Roessler, Peter, Dr.**
**Elsterstrasse 15**
**D-506 Bensberg 1(DE)**

(54) **N-(omega-Chloralkanoyl)-N'-trifluormethylphenyl-harnstoffe, Verfahren zu ihrer Herstellung und ihre Verwendung als Pflanzenschutzmittel.**

(57) Die Erfindung betrifft neue N-(ω-Chloralkanoyl)-N'trifluormethylphenyl- harnstoffe, mehrere Verfahren zu ihrer Herstellung sowie ihre Verwendung als Pflanzenschutzmittel. Die Verbindungen der Formel, in welcher R und n

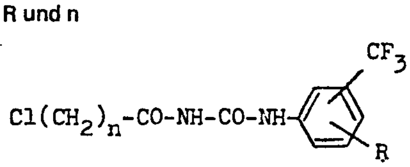
(I)

die in der Beschreibung angegebene Bedeutung besitzen, werden erhalten, wenn man ω-Chloralkanoylisocyanate mit Trifluormethyl- anilinen in Gegenwart eines inerten Lösungs- oder Verdünnungsmittels umsetzt. Daneben gibt es noch andere Herstellungsverfahren. Die erfindungsgemäßen Wirkstoffe können angewandt werden gegen parasitäre Pilze und Bakterien, die oberirdische Pflanzenteile befallen oder die Pflanzen vom Boden her angreifen, sowie gegen samenübertragbare Krankheitserreger. Die Wirkstoffe eignen sich bei guter Pflanzenverträglichkeit und günstiger Warmblütertoxizität ferner zur Bekämpfung von tierischen Schädlingen, insbesondere Insekten, die in der Landwirtschaft, in Forsten, im Vorrats- und Materialschutz sowie auf dem Hygienesektor vorkommen.

EP 0 003 766 A1

Croydon Printing Company Ltd.

BAYER AKTIENGESELLSCHAFT
Zentralbereich
Patente, Marken und Lizenzen

5090 Leverkusen, Bayerwerk
Slr/bc
I b

**N-($\omega$-Chloralkanoyl)-N'-trifluormethylphenyl-harnstoffe, Verfahren zu ihrer Herstellung und ihre Verwendung als Pflanzenschutzmittel**

Die Erfindung betrifft neue N-($\omega$-Chloralkanoyl)-N'-trifluormethylphenyl-harnstoffe, mehrere Verfahren zu ihrer Herstellung sowie ihre Verwendung als Pflanzenschutzmittel.

Es ist bekannt, daß N-Halogenalkylthio-phthalimide, wie z.B. N-Trichlormethylthio-tetrahydrophthalimid, fungizide Eigenschaften aufweisen und als Handelsprodukte im Pflanzenschutz weltweit verwendet werden (vgl. R. Wegler, Chemie der Pflanzenschutz- und Schädlingsbekämpfungsmittel, Band 2, S. 108, Springer-Verlag Berlin/Heidelberg/New York, 1970).
Die Wirkung solcher Verbindungen ist jedoch, insbesondere bei niedrigen Aufwandmengen, nicht immer ganz befriedigend.

Wirkstoffe, die die Metamorphose von Arthropoden hemmen, sind erst seit jüngerer Zeit im Pflanzenschutz von Interesse. Zu nennen ist hier z.B. das 2,2-Dimethyl-6-methoxybenzopyran (Chem.Eng. News 54, 19-20 (1976)).

Es wurden die neuen N-(ω-Chloralkanoyl)-N'-trifluormethylphenyl-harnstoffe der Formel

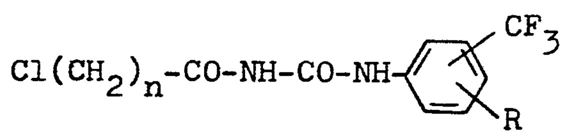

(I)

in welcher

R für Halogenalkyl oder Halogen steht und außerdem noch für Wasserstoff stehen kann, wenn n für die Zahl 2 steht, und

n für die Zahlen 1 oder 2 steht,

gefunden. Diese neuen Verbindungen zeichnen sich durch starke fungizide Eigenschaften aus; ferner hemmen sie die Entwicklung von Arthropoden. Sie sind daher als Pflanzenschutzmittel von Interesse.

Weiterhin wurde gefunden, daß die neuen N-(ω-Chloralkanoyl)-N'-trifluormethylphenyl-harnstoffe der Formel (I) erhalten werden, wenn man

a) ω-Chloralkanoylisocyanate der Formel

$$Cl-(CH_2)_n-CO-NCO \quad (II)$$

worin

n die oben angegebene Bedeutung hat,

mit Trifluormethyl-anilinen der Formel

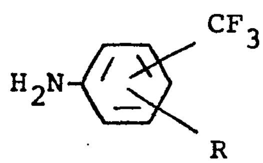

(III)

in welcher
R die oben angegebene Bedeutung hat,

gegebenenfalls in Gegenwart eines inerten Lösungs- oder Verdünnungsmittels umsetzt, oder

b) ω-Chloralkanoyl-chloride bzw. die entsprechenden Anhydride der Formeln

$$Cl-(CH_2)_n-CO-Cl \qquad (IV)$$

bzw.

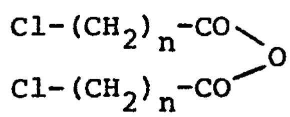

(V)

worin
n die oben angegebene Bedeutung hat,

mit Trifluormethylphenyl-harnstoffen der Formel

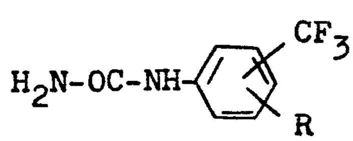

(VI)

in welcher
R die oben angegebene Bedeutung hat,

gegebenenfalls in Gegenwart eines Säureakzeptors und gegebenenfalls in Gegenwart eines inerten Lösungs- oder Verdünnungsmittels umsetzt, oder

c) ω-Chloralkanoyl-amine der Formel

$$Cl-(CH_2)_n-CO-NH_2 \qquad (VII)$$

in welcher

n die oben angegebene Bedeutung hat,

mit Trifluormethylphenylisocyanaten der Formel

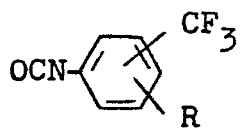

(VIII)

in welcher

R die oben angegebene Bedeutung hat,

gegebenenfalls in Gegenwart eines inerten Lösungs- oder Verdünnungsmittels und gegebenenfalls unter Verwendung von Katalysatoren umsetzt.

Überraschenderweise besitzen die erfindungsgemäßen N-(ω-Chloralkanoyl)-N'-trifluormethylphenyl-harnstoffe eine wesentlich bessere fungizide Wirkung als die aus dem Stand der Technik bekannten N-Halogenalkylthio-phthalimide gleicher Wirkungsrichtung. Von Interesse ist weiterhin die die Entwicklung von Arthropoden hemmende Wirkung. Die erfindungsgemäßen Stoffe stellen somit eine Bereicherung der Technik dar.

Verwendet man nach Verfahren (a) Chloracetylisocyanat und 2-Trifluormethyl-4-chlor-anilin, nach Verfahren (b) ω-Chlorpropionylchlorid und 4-Trifluormethylphenylharnstoff in Gegenwart einer Base und nach Verfahren

(c) Chloracetamid und 3,5-Bis-trifluormethyl-phenylisocyanat als Ausgangsstoffe, so kann der Reaktionsverlauf durch die folgenden Formelschemata wiedergegeben werden:

a)

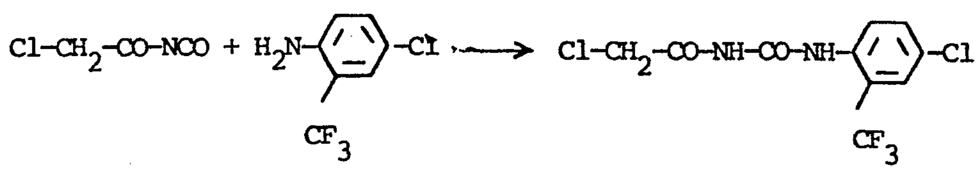

b)

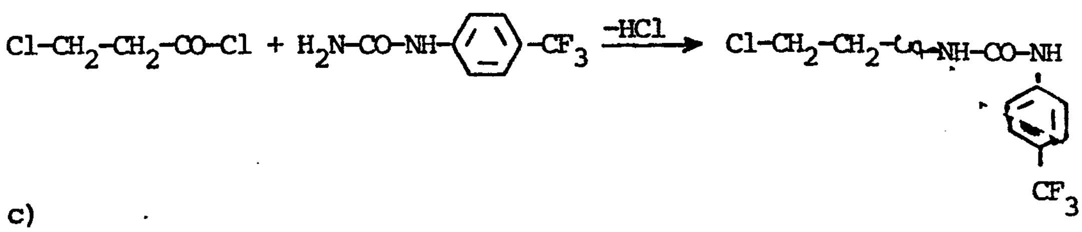


c)

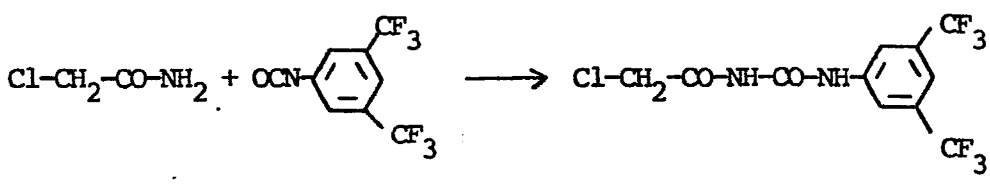

Die zu verwendenden Ausgangsstoffe sind durch die Formeln (II) bis (VIII) allgemein definiert. Vorzugsweise steht darin jedoch R für Trifluormethyl oder Chlor und, wenn der Index n für die Zahl 2 steht, vorzugsweise für Wasserstoff.

Die als Ausgangsstoffe zu verwendenden ω-Chloralkanoylisocyanate (II) sind bekannt oder nach literaturbekannten Verfahren herstellbar (vgl. J.Org.Chem. 27 (1962), 3742 und J.Org.Chem. 28 (1963), 1805). So läßt sich z.B. Chloracetamid mit Oxalylchlorid bei 80°C umsetzen, nach einer

Reaktionszeit von 24 Stunden erhält man Chloracetylisocyanat neben Kohlenmonoxid und Chlorwasserstoff in einer Ausbeute von 64 % der Theorie.

Als Beispiele seien genannt:
Chloracetylisocyanat und ω-Chlor-propionylisocyanat.

Die weiterhin als Ausgangsstoffe zu verwendenden Trifluormethyl-aniline der Formel (III) sind ebenfalls bekannt oder nach literaturbekannten Verfahren herstellbar (vgl. Adv. Fluorine Chem. 6 (1970), 1). So läßt sich z.B. o-Nitrotoluol an der Methylgruppe chlorieren zum 1-Trichlormethyl-2-nitrobenzol, die Umsetzung mit Fluorwasserstoff führt zur entsprechenden Trifluormethyl-Verbindung, und die Reduktion der letzteren ergibt in üblicher Weise das o-Trifluormethyl-anilin. Die Aniline der Formel (III) können nach allgemein üblichen Methoden (vgl. Indian J.Appl.Chem. 35, 129-130 (1972)) in die Trifluormethylphenyl-harnstoffe der Formel (V) bzw. in die Trifluormethylphenylisocyanate der Formel (VIII) umgewandelt werden, z.B. durch Umsetzung mit Alkalicyanaten bzw. mit Phosgen.

Als Beispiele seien genannt:

2-Trifluormethyl-, 4-Trifluormethyl-, 2,3-Bis-trifluormethyl-, 2,4-Bis-trifluormethyl-, 2,5-Bis-trifluormethyl-, 2,6-Bis-trifluormethyl-, 3,4-Bis-trifluormethyl-, 3,5-Bis-trifluormethyl-, 2-Trifluormethyl-3-chlor-, 2-Trifluormethyl-4-chlor-, 2-Trifluormethyl-5-chlor-, 2-Trifluormethyl-6-chlor-, 3-Trifluormethyl-2-chlor-, 3-Trifluormethyl-4-chlor-, 3-Trifluormethyl-5-chlor, 3-Trifluormethyl-6-chlor-, 4-Trifluormethyl-2-chlor-, 4-Trifluormethyl-3-chlor-anilin bzw. -phenylharnstoff bzw. -phenylisocyanat.

Die ferner als Ausgangsstoffe zu verwendenen ω-Chloralkanoylchloride der Formel (IV) bzw. die entsprechenden Amide der Formel (VII) sind als technische Zwischenprodukte allgemein bekannt.

Als Beispiele seien genannt:

Chloressigsäure-chlorid, -anhydrid und -amid, ω-Chlorpropionsäure-chlorid, -anhydrid und -amid.

Die Verfahren zur Herstellung der erfindungsgemäßen N-(ω-Chloralkanoyl)-N'-trifluormethylphenyl-harnstoffe werden bevorzugt unter Verwendung geeigneter Lösungs- oder Verdünnungsmittel durchgeführt. Als solche kommen praktisch alle inerten organischen Solventien in Frage. Hierzu gehören insbesondere aliphatische und aromatische, gegebenenfalls chlorierte, Kohlenwasserstoffe, wie Benzin, Benzol, Toluol, Xylol, Methylenchlorid, Chloroform, Tetrachlorkohlenstoff, Chlorbenzol und o-Dichlorbenzol, Äther, wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Die Reaktionstemperatur kann bei allen Herstellungsverfahren innerhalb eines größeren Bereichs variiert werden. Im allgemeinen arbeitet man zwischen 0 und 150°C, vorzugsweise bei 20 bis 120°C. Bei der Verfahrensvariante a) wird insbesondere ein Temperaturbereich zwischen 30 und 80°C bevorzugt.

Die Umsetzungen werden im allgemeinen bei Normaldruck durchgeführt.

Als Säurebinder können bei dem Verfahren b) übliche anorganische oder organische Säurebinder verwendet werden. Hierzu gehören vorzugsweise die Alkalihydroxide und Alkalicarbonate, wie z.B. Natriumhydroxid und Kaliumcarbonat, ferner tertäre Amine, wie z.B. Triäthylamin oder Pyridin.

Bei der Verfahrensvariante c) können Katalysatoren Verwendung finden. Zu nennen sind hier organische Basen, wie z.B. Triäthylamin, oder Metallsalze, wie z.B. Zinn-II-Verbindungen.

Zur Durchführung der Herstellungsverfahren setzt man die Ausgangsverbindungen vorzugsweise in äquimolaren Mengen ein. Ein Überschuß der einen oder anderen Reaktionskomponente bringt keine wesentlichen Vorteile. Im allgemeinen gibt man die Reaktionspartner in einem der angegebenen Lösungsmittel zusammen und fügt gegebenenfalls eine Hilfsbase hinzu. Nach ein- oder mehrstündigem Rühren im angegebenen Temperaturbereich und anschließendem Abkühlen auf Raumtemperatur können die kristallin anfallenden Produkte durch Filtration isoliert werden. Die neuen Produkte werden durch ihren Schmelzpunkt charakterisiert.

Die erfindungsgemäßen Wirkstoffe weisen eine gute fungitoxische Wirkung auf. Sie schädigen Kulturpflanzen in den zur Bekämpfung von Pilzen notwendigen Konzentrationen nicht. Aus diesen Gründen sind sie für den Gebrauch als

Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe können angewandt werden gegen parasitäre Pilze und Bakterien, die oberirdische Pflanzenteile befallen oder die Pflanzen vom Boden her angreifen, sowie gegen samenübertragbare Krankheitserreger.

Die gute Pflanzenverträglichkeit erlaubt eine Anwendung gegen pilzliche Pflanzenkrankheiten durch Behandlung der stehenden Kulturpflanze oder einzelner Teile von ihr oder des Saatgutes oder auch des Kulturbodens. Die Wirkstoffe sind besonders wirksam gegen Venturia-Arten und gegen solche Pilze, die Getreidekulturen schädigen.

Die Wirkstoffe eignen sich bei guter Pflanzenverträglichkeit und günstiger Warmblütertoxizität ferner zur Bekämpfung von tierischen Schädligen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.

Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp..
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp..
Aus der Ordnung der Homoptera z.B. Aleurodes brassicae, Bemisia tabaci, Trialeurodes vaporariorum, Aphis goxxypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cinciticeps, Lecanium corni, Saissetia cleae,

Laodelphax striatellus, Nilaparvata lugens, Aonidiella aurantii, Aspidiotus hederae, Pseudococcus spp., Psylla spp..

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carbpocapsa pomonella, Pieris spp., Chilo spp., Pyrausta nubilalis, Ephestia kuehniella, Galleria mellonella, Cacoecia podana, Capua reticulana, Choristoneura fumiferana, Clysis ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp.,Psylliodes chrysocephala, Epilachna varivestis, Atomaria spp., Cryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctux spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp..

Aus der Ordnung der Diptera z.B. Aëdes spp., Anopheles spp., Culex spp., Drosophila melanogaster, Musca spp., Fannia spp., Calliphora erythrocephala, Lucilia spp., Chrysomyia spp., Cuterebra spp., Hyppoboxca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..

Wie schon erwähnt, hemmen die erfindungsgemäßen Verbindungen die Entwicklung von Gliederfüßlern (Arthrophoden).

Durch die im Beispielteil angegebenen Versuche wird die arthropodenmethamorphosehemmende Wirkung der erfindungsgemäßen Verbindungen gezeigt, ohne eine Beschränkung hinsichtlich der Wirkugnsbreite dieser Verbindungen vornehmen zu wollen.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierenen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glykol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykoläther, Alkylsulfonate, Alkylsulfate, Arylsulfonate sowie Eiweißhydrolysate; als Dispergiermittel: z.B. Lignin, Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die Anwendung der erfindungsgemäßen Wirkstoffe erfolgt in Form ihrer handelsüblichen Formulierenen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-%, liegen.

Die Anwendung geschieht in einer den Anwendungsformen angepaßten üblichen Weise.

Bei der Beheizung kommen im allgemeinen je Kilogramm Saatgut Wirkstoffmengen von 10 mg bis 10 g, vorzugsweise 100 mg bis 3 g zur Anwendung. Bei der Bodenbehandlung, die ganzflächig, streifenförmig oder punktförmig durchgeführt werden kann, werden am Ort der erwarteten Wirkung Wirkstoffkonzentrationen von 1 bis 1000 g Wirkstoff je $m^3$ Boden benötigt, vorzugsweise 10 bis 200 g pro $m^3$.

Beispiel A:

Myzelwachstums-Test

Verwendeter Nährboden:

| | | |
|---|---|---|
| 20 | Gew.-Teile | Agar-Agar |
| 200 | Gew.-Teile | Kartoffeldekokt |
| 5 | Gew.-Teile | Malz |
| 15 | Gew.-Teile | Dixtrose |
| 5 | Gew.-Teile | Pepton |
| 2 | Gew.-Teile | Dinatriumphosphat |
| 0,3 | Gew.-Teile | Calciumnitrat |

Verhältnis von Lösungsmittelgemisch zum Nährboden:

| | | |
|---|---|---|
| 2 | Gew.-Teile | Lösungsmittelgemisch |
| 100 | Gew.-Teile | Agarnährboden |

Zusammensetzung Lösungsmitgelgemisch:

| | | |
|---|---|---|
| 0,19 | Gew.-Teile | Dimethylformamid |
| 0,01 | Gew.-Teile | Emulgator (Alkyl-aryl-polyglykoläther) |
| 1,80 | Gew.-Teile | Wasser |
| 2 | Gew.-Teile | Lösungsmittelgemisch |

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den unten angegebenen Pilzarten beimpft und bei etwa 21°C inkubiert.

Die Auswertung erfolgt je nach der Wachstumsgeschwindigkeit der Pilze nach 4 bis 10 Tagen. Bei der Auswertung wird das radiale Myzelwachstum auf den behandelten Nährböden mit dem Wachstum auf dem Kontrollnährboden verglichen. Die Bonitierung des Pilzwachstums geschieht mit Kennzahlen, wobei

1 kein Pilzwachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

bedeuten. Die Versuchsauswertung ergab, daß z.B. die Verbindung gemäß Herstellungsbeispiel 1 eine gute Wirkung gegen die Pilzarten Rhizoctonia solani, Cochliobolus miyabeanus, Pyricularia oryzae, Helminthosporium gramineum, Mycosphaerella musicola, Phytophthora cactorum und Pellicularia sasakii aufweist.

Beispiel B:

Fusicladium-Test (Apfelschorf)/Protektiv

Lösungsmittel: 4,7 Gew.-Teile Aceton
Emulgator: 0,3 Gew.-Teile Alkyl-aryl-polyglykoläther
Wasser: 95 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4 bis 6 Blattstadium befinden, bis zur Tropfnässe. Die Pflanzen verbleiben 24 Stunden bei 20$^{o}$C und einer relativen Luftfeuchtigkeit von 70 % im Gewächshaus. Anschließend werden sie mit einer wäßrigen Konidiensuspension des Apfelschorferregers (Fusicladium dendriticum Fuck.) inokuliert und 18 Stunden lang in einer Feuchtkammer bei 18 bis 20$^{o}$C und 100 % relativer Luftfeuchtigkeit inkubiert.

Die Pflanzen kommen dann erneut für 14 Tage ins Gewächshaus.

15 Tage nach der Inokulation wird der Befall der Sämlinge in % der unbehandelten, jedoch ebenfalls inokulierten Kontrollpflanzen bestimmt.

0 % bedeutet keinen Befall, 100 % bedeutet, daß der Befall genau so hoch ist wie bei den Kontrollpflanzen.

Die Versuchsauswertung ergab, daß z.B. die Verbindung gemäß Herstellungsbeispiel 1 eine dem beim Stand der Technik angegebenen Vergleichspräparat überlegene Wirkung aufweist.

Bei den folgenden Beispielen zur entwicklungshemmenden Wirkung der Wirkstoffe werden während der gesamten angegebenen Entwicklung der Testtiere die morphologischen Veränderungen, wie zur Hälfte verpuppte Tiere, unvollständig geschlüpfte Larven oder Raupen, defekte Flügel, pupale Kutikula bei Imagines etc., als Mißbildungen gewertet. Die Summe der morphologischen Mißbildungen, zusammen mit den während des Häutungsgeschehens oder der Metamorphose abgetöteten Tieren, wird in Prozent der Gesamtzahl der eingesetzten Versuchstiere bestimmt.

Beispiel C:

Entwicklungshemmende Wirkung / Aedes aegypti-Test

Testtiere: Aedes aegypti (Larven im 3. Entwicklungsstadium)

Zahl der Testtiere: 20 Stück

Lösungsmittel: 10 Gewichtsteile Aceton

Emulgator: 1 Gewichtsteil Alkylarylpolyglykoläther

Zur Herstellung einer zweckmäßigen Wirkstoffzubereitung vermischt man 2 Gewichtsteile Wirkstoff mit der angegebenen Menge Lösungsmittel, Emulgator und so viel Wasser, daß eine 100 ppm-haltige Mischung entsteht, die mit Wasser auf die gewünschte Konzentration verdünnt wird.

Die Testtiere werden in 90 ml einer Wirkstoffzubereitung der gewünschten Konzentration eingesetzt und bis zum Schlüpfen der Imago beobachtet. Zur Kontrolle werden Testtiere in ein Lösungsmittel- und Emulgator-Wassergemisch der entsprechenden Konzentration gebracht und bis zum Schlüpfen der Imago beobachtet.

Bei diesem Test zeigen z.B. die folgenden Verbindungen der Herstellungsbeispiele überlegene Wirkung gegenüber dem Stand der Technik.

Verbindung gemäß Herstellungsbeispiel 1.

Beispiel D:

Entwicklungshemmende Wirkung / Laphygma-Raupen-Test

| | |
|---|---|
| Testtiere: | Laphygma frugiperda (Raupen) |
| Futter: | 1 cm dicke Scheibe von 3 cm Durchmesser luftangetrocknetes Kunstfutter aus Bohnenkernschrot, Hefe, Vitaminmischung, Blattpulver, Agar und Konservierungsstoff |
| Lösungsmittel: | 10 Gewichtsteile Aceton |
| Emulgator: | 1 Gewichtsteil Alkylarylpolyglykoläther |

Zur Herstellung einer zweckmäßigen Wirkstoffzubereitung vermischt man 2 Gewichtsteile Wirkstoff mit der angegebenen Menge Lösungsmittel, Emulgator und soviel Wasser, daß eine 1 %ige Mischung entsteht, die mit Wasser auf die gewünschte Konzentration verdünnt wird.

Jeweils ein Testtier wird auf eine mit 1,5 ml Wirkstoffzubereitung der gewünschten Konzentration angefeuchtete Futterscheibe gesetzt und bis zum Schlüpfen der Imago beobachtet.

Zur Kontrolle wird je ein Testtier auf eine mit 1,5 ml Lösungsmittel- und Emulgator-Wassergemisch der entsprechenden Konzentration angefeuchtete Futterscheibe gesetzt und bis zum Schlüpfen der Imago beobachtet.

Bei diesem Test zeigen z.B. die folgenden Verbindungen der Herstellungsbeispiele überlegene Wirkung gegenüber dem Stand der Technik.

Verbindung gemäß Herstellungsbeispiel 1.

Herstellungsbeispiele

Beispiel 1

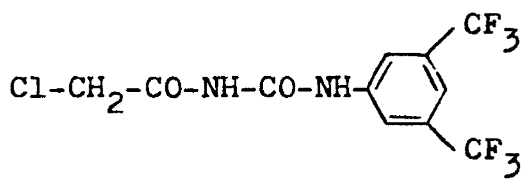

22,9 g (0,1 Mol) 3,5-Bis-trifluormethyl-anilin werden in 150 ccm trockenem Toluol gelöst. Dazu fügt man 11,9 g (0,1 Mol) Chloracetylisocyanat in 50 ccm trockenem Toluol. Die Mischung wird 1 Stunde bei 50°C gerührt und dann auf Raumtemperatur abgekühlt. Das ausgefallene Produkt wird abgesaugt und mit Toluol/Ligroin 1:2 nachgewaschen und anschließend getrocknet. Man erhält so 26 g (74,5 % der Theorie) N-(3,5-Bis-trifluormethylphenyl)-N'-(chloracetyl)-harnstoff mit einem Schmelzpunkt von 170°C.

Beispiel 2

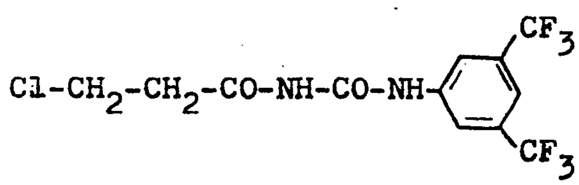

11,45 g (0,05 Mol) 3,5-Bis-trifluormethyl-anilin werden in 80 ccm trockenem Toluol gelöst und mit 6,7 g (0,05 Mol) ß-Chlorpropionylisocyanat in 20 ccm trockenem Toluol versetzt. Der Ansatz wird 1 Stunde bei 60°C gerührt, das Lösungsmittel im Vakuum abdestilliert und der Rückstand in

Petroläther aufgenommen. Das kristalline Produkt wird abgesaugt und getrocknet. Nam erhält 14,5 g (80,5 % der Theorie) an N-(3,5-Bis-trifluormethyl-phenyl)-N'-(ß-chlorpropionyl)-harnstoff, der bei $175^{\circ}$C schmilzt.

Die Reinheit und Identität der Produkte wurde durch Elementaranalyse und durch das NMR-Spektrum sichergestellt. Die Ausbeuten sind nicht optimiert.

In entsprechender Weise können die folgenden Verbindungen der allgemeinen Formel

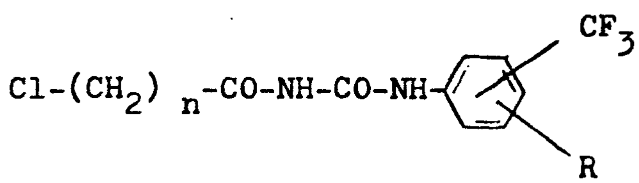

hergestellt werden:

| Beispiel Nr. | n | $CF_3$-Stellung | R | Schmelzpunkt (°C) | Ausbeute (% der Theorie) |
|---|---|---|---|---|---|
| 3 | 1 | 3 | 4-$CF_3$ | 113 | 77,5 |
| 4 | 1 | 2 | 4-$CF_3$ | 156 | 57,5 |
| 5 | 1 | 3 | 4-Cl | 162 | 76 |
| 6 | 1 | 5 | 2-Cl | 17o | 7o |
| 7 | 1 | 2 | 4-Cl | 147 | 66,5 |
| 8 | 1 | 4 | 2-Cl | 17o | 66,5 |
| 9 | 2 | 3 | H | 127 | 55 |
| 1o | 2 | 4 | H | 172 | 83,5 |
| 11 | 2 | 3 | 4-$CF_3$ | 161 | 82,5 |
| 12 | 2 | 2 | 4-$CF_3$ | 176 | 94 |

| Beispiel Nr. | n | $CF_3$-Stellung | R | Schmelzpunkt (°C) | Ausbeute (% der Theorie) |
|---|---|---|---|---|---|
| 13 | 2 | 3 | 4-Cl | 178 | 59 |
| 14 | 2 | 5 | 2-Cl | 157 | 65 |
| 15 | 2 | 2 | 4-Cl | 183 | 94 |
| 16 | 2 | 4 | 2-Cl | 198 | 89,5 |
| 17 | 2 | 4 | 3-Cl | 167 | 85 |

Die Ausbeuten wurden nicht optimiert.

Die Herstellung der beanspruchten Produkte kann auch durch Umsetzung von Trifluormethyl-phenyl-harnstoff mit den entsprechenden Anhydriden oder mit den entsprechenden Säurehalogeniden in Gegenwart einer Base vorgenommen werden. Eine weitere Herstellungsvariante ist die Umsetzung der ω-Halogensäureamide mit den entsprechenden Trifluormethyl-phenylisocyanaten.

Vorprodukte:

$Cl-CH_2-CO-NCO$

Die Verbindung wurde aus Chloracetamid und Oxalylchlorid nach Literaturangaben hergestellt (J.Org.Chem. 27, 3742 (1962) und J.Org.Chem. 28, 1805 (1963)). Kp 43°C/13 mm Hg.

In entsprechender Weise wurde erhalten:

$Cl-CH_2-CH_2-CO-NCO$ Kp 47°C/5 mm Hg

Patentansprüche

1) N-(ω-Chloralkanoyl)-N'-trifluormethylphenyl-harnstoffe der Formel

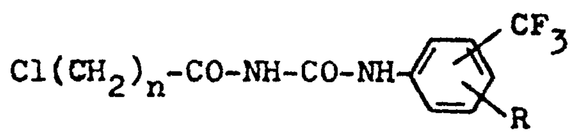

(I)

in welcher

R für Halogenalkyl oder Halogen steht und außerdem noch für Wasserstoff stehen kann, wenn n für die Zahl 2 steht, und

n für die Zahlen 1 oder 2 steht.

2) Verfahren zur Herstellung von N-(ω-Chloralkanoyl)-N'-trifluormethylphenyl-harnstoffen, dadurch gekennzeichnet, daß man

a) ω-Chloralkanoylisocyanate der Formel

$$Cl-(CH_2)_n-CO-NCO \qquad (II)$$

worin

n die in Anspruch 1 angegebene Bedeutung hat,

mit Trifluormethyl-anilinen der Formel

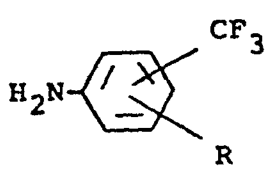

(III)

in welcher

R die in Anspruch 1 angegebene Bedeutung hat,

gegebenenfalls in Gegenwart eines inerten Lösungs- oder Verdünnungsmittels umsetzt, oder

b) ω-Chloralkanoyl-chloride bzw. die entsprechenden Anhydride der Formeln

$$Cl-(CH_2)_n-CO-Cl \qquad (IV)$$

bzw.

$$\begin{matrix} Cl-(CH_2)_n-CO \\ Cl-(CH_2)_n-CO \end{matrix} \!\!> O \qquad (V)$$

worin

n die in Anspruch 1 angegebene Bedeutung hat,

mit Trifluormethylphenyl-harnstoffen der Formel

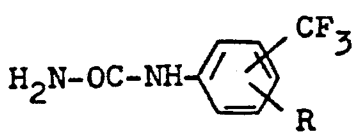

(VI)

in welcher

R die in Anspruch 1 angegebene Bedeutung hat,

gegebenenfalls in Gegenwart eines Säureakzeptors und gegebenenfalls in Gegenwart eines inerten Lösungs- oder Verdünnungsmittels umsetzt, oder

c) ω-Chloralkanoyl-amine der Formel

$$Cl-(CH_2)_n-CO-NH_2 \qquad (VII)$$

in welcher
n die in Anspruch 1 angegebene Bedeutung hat,

mit Trifluormethylphenylisocyanaten der Formel

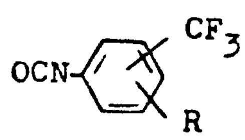

(VIII)

in welcher
R die in Anspruch 1 angegebene Bedeutung hat,

gegebenenfalls in Gegenwart eines inerten Lösungs- oder Verdünnungsmittels und gegebenenfalls unter Verwendung von Katalysatoren umsetzt.

3) Pflanzenschutzmittel, gekennzeichnet durch einen Gehalt an mindestens einem N-(ω-Chloralkanoyl)-N'-trifluormethylphenylharnstoff gemäß Anspruch 1.

4) Fungizide und die Entwicklung von Arthropoden hemmende Mittel, gekennzeichnet durch einen Gehalt an mindestens einem N-(ω-Chloralkanoyl)-N'-trifluormethylphenylharnstoff gemäß Anspruch 1.

5) Verfahren zur Bekämpfung von Pilzen und zur Entwicklungshemmung von Arthropoden, dadurch gekennzeichnet, daß man N-(ω-Chloralkanoyl)-N'-trifluormethylphenylharnstoffe gemäß Anspruch 1 auf Pilze oder Arthropoden oder ihren Lebensraum einwirken läßt.

6) Verwendung von N-(ω-Chloralkanoyl)-N'-trifluormethylphenylharnstoffen gemäß Anspruch 1 zur Bekämpfung von Pilzen und zur Entwicklungshemmung von Arthropoden.

7) Verfahren zur Herstellung von fungizid wirksamen und die Entwicklung von Arthropoden hemmenden Mitteln, dadurch gekennzeichnet, daß man N-(ω-Chloralkanoyl)-N'-trifluormethylphenylharnstoffe gemäß Anspruch 1 mit Streckmitteln und/oder oberflächenaktiven Mitteln vermischt.

0003766

Europäisches Patentamt

# EUROPÄISCHER RECHERCHENBERICHT

Nummer der Anmeldung
EP 79 10 0340

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US - A - 3 318 947 (SPEZIALE & SMITH)<br>* Spalte 2, Zeilen 33-60 *<br>---- | 1 |

KLASSIFIKATION DER ANMELDUNG (Int.Cl.²)

C 07 C 127/22
A 01 N 9/20

RECHERCHIERTE SACHGEBIETE (Int. Cl.²)

C 07 C 127/22

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 07-05-1979 | GAUTIER |